# FASCICULE DE BREVET EUROPEEN

(11) **EP 4 526 047 B1**
(45) Date de publication et mention de la délivrance du brevet: **08.07.2026**
(21) Numéro de dépôt: 23729810.4
(22) Date de dépôt: 15.05.2023
(51) Int. Cl.: B05B 12/08

(54) **PROCEDE ET DISPOSITIF D'ANALYSE D'UN DISPOSITIF DE PULVERISATION DE PRODUIT FLUIDE PHARMACEUTIQUE**
VERFAHREN UND VORRICHTUNG ZUR ANALYSE EINER VORRICHTUNG ZUM SPRÜHEN EINES FLÜSSIGEN PHARMAZEUTISCHEN PRODUKTS
METHOD AND DEVICE FOR ANALYSING A DEVICE FOR SPRAYING A FLUID PHARMACEUTICAL PRODUCT

(30) Priorité: 16.05.2022 FR 2204608
(43) Date de publication de la demande: 26.03.2025
(73) Titulaire: APTAR France SAS, 27110 Le Neubourg (FR)
(72) Inventeur: GRANJON, Guillaume, 27400 LA VACHERIE (FR)
(74) Mandataire: CAPRI
(86) Numéro de dépôt international: PCT/FR2023/050690
(87) Numéro de publication internationale: WO 2023/222969

(56) Documents cités:
- DE-A1- 102017 006 969
- JP-A- H0 599 802
- JP-A- S54 127 347
- JP-A- S54 127 348

## Description

La présente invention concerne un dispositif et un procédé d'analyse de spray généré par un dispositif de pulvérisation de produit fluide pharmaceutique.

Les dispositifs de pulvérisation de produit fluide pharmaceutique sont bien connus. Ils comportent généralement une tête de pulvérisation pourvue d'un orifice de pulvérisation, assemblée sur un réservoir contenant le produit fluide à distribuer. En particulier dans des applications de pulvérisation nasale, l'efficacité thérapeutique du produit fluide pulvérisé peut dépendre des propriétés du spray généré lors de l'actionnement du dispositif. De manière connue, à la fin de la chaîne de montage, c'est-à-dire lorsque le dispositif de pulvérisation est assemblé, et juste avant d'être expédié chez le fabriquant du produit fluide pharmaceutique pour y être assemblé sur un réservoir correspondant, un certain nombre d'échantillons de dispositifs assemblés sont testés en laboratoire pour vérifier si les propriétés du spray correspondent au cahier des charges prédéfini.

Un inconvénient de ce système est qu'il concerne des dispositifs assemblés, et donc destructeur de ces dispositifs qui ne pourront plus, après avoir été testés, être livrés au client.

De plus, ce système impose une vérification humaine des dispositifs testés, et n'est donc pas totalement automatisable.

Pour surmonter cet inconvénient, le document WO2018130791 propose la visualisation par strioscopie d'un flux d'air comprimé chaud ou froid envoyé à travers une tête de pulvérisation. Cette méthode permet d'évaluer l'angle du spray mais pas sa géométrie ni sa symétrie. Ceci a de plus l'inconvénient d'avoir à prévoir un banc strioscopique, relativement complexe et coûteux, et difficilement adaptable dans une chaine de montage d'un dispositif de pulvérisation de produit fluide, et implique donc soit des tests aléatoires sur une partie seulement des dispositifs fabriqués, soit un ralentissement de la chaine de montage, généralement peu souhaitable.

Le document EP3047912 décrit un système et un procédé de visualisation utilisant la détection d'une différence de température entre un flux de gaz chauffé traversant une tête de pulvérisation et une zone de réception à température ambiante, pour déterminer la conformité de ladite tête de pulvérisation. Cette solution nécessite des moyens de chauffage et l'utilisation d'une caméra thermique, ce qui rend l'ensemble complexe et coûteux à utiliser.

Le document WO2022003294 décrit également un système et un procédé de visualisation utilisant la détection d'une différence de température avec des moyens de détection thermosensibles.

Les documents JPH0599802 et JPS54127347 décrivent d'autres dispositifs de l'état de la technique.

La présente invention a pour but de surmonter les inconvénients susmentionnés.

En particulier, la présente invention a pour but de fournir un dispositif et un procédé d'analyse d'un dispositif de pulvérisation de produit fluide pharmaceutique qui soit non destructeur des dispositifs testés.

La présente invention a également pour but de fournir un dispositif et un procédé d'analyse qui soit automatisé en grande partie.

La présente invention a aussi pour but de fournir un dispositif et un procédé d'analyse qui permette de tester 100% des dispositifs de pulvérisation, sans ralentir la chaine de montage de manière substantielle.

La présente invention a également pour but de fournir un dispositif et un procédé d'analyse qui n'utilise ni moyens de chauffage, ni caméra thermique, ni moyens de détection thermosensibles.

La présente invention a également pour but de fournir un dispositif et un procédé d'analyse qui soit simple et/ou peu coûteux à fabriquer, à assembler et à utiliser.

La présente a donc pour objet un procédé d'analyse d'un dispositif de pulvérisation de produit fluide pharmaceutique, comprenant les étapes suivantes :
- fournir une tête de pulvérisation d'un dispositif de pulvérisation de produit fluide pharmaceutique, ladite tête de pulvérisation comportant un orifice de pulvérisation,
- fournir une zone de réception formant un réservoir dont le fond est formé par une plaque transparente derrière laquelle est disposée une caméra, ledit réservoir contenant un ferrofluide,
- fournir, sous ladite plaque transparente, un ensemble d'aimants générant une charge magnétique pour contraindre ledit ferrofluide à recouvrir ladite plaque transparente,
- faire passer un flux de gaz à travers ledit orifice de pulvérisation de ladite tête de pulvérisation, et l'envoyer sur ladite zone de réception, ledit ferrofluide contacté par ledit flux de gaz étant déplacé sur ladite plaque transparente pour former une zone d'impact,
- visualiser ladite zone d'impact avec ladite caméra, et
- analyser avec des moyens d'analyse ladite visualisation de ladite zone d'impact pour déterminer si ladite zone d'impact est conforme ou non conforme à des spécifications prédéterminées.

Avantageusement, ledit flux de gaz est un flux de gaz comprimé.

Avantageusement, ledit flux de gaz est un flux d'air comprimé.

Avantageusement, ladite étape d'analyser comprend de déterminer la géométrie, notamment la symétrie, de la zone d'impact dudit flux de gaz sur ladite zone de réception.

Avantageusement, lesdites spécifications prédéterminées comprennent une étendue planaire prédéterminée de la zone d'impact dudit flux de gaz sur ladite zone de réception, de sorte que les têtes de pulvérisation pour lesquelles ladite étendue planaire est similaire à ladite étendue planaire prédéterminée sont classées conformes, et les têtes de pulvérisation pour lesquelles ladite étendue planaire est différente de ladite étendue planaire prédéterminée sont classés non conformes.

Avantageusement, un cycle d'utilisation comprend les étapes suivantes:
- activer ledit ensemble d'aimants pour générer une charge magnétique pour contraindre ledit ferrofluide à recouvrir ladite plaque transparente,
- générer ledit flux de gaz et l'envoyer à travers ladite tête de pulvérisation sur ladite zone de réception, le ferrofluide contacté par ledit flux de gaz étant repoussé vers les bords de ladite zone de réception pour former une zone d'impact sur ladite plaque transparente,
- visualiser ladite zone d'impact en prenant une image de ladite plaque transparente au moyen de ladite caméra.

La présente a également pour objet un dispositif d'analyse d'un dispositif de pulvérisation de produit fluide pharmaceutique, comprenant :
- une tête de pulvérisation d'un dispositif de pulvérisation de produit fluide pharmaceutique, ladite tête de pulvérisation comportant un orifice de pulvérisation,
- une zone de réception formant un réservoir dont le fond est formé par une plaque transparente, ledit réservoir contenant un ferrofluide,
- un ensemble d'aimants générant une charge magnétique pour contraindre ledit ferrofluide à recouvrir ladite plaque transparente,
- des moyens de génération d'un flux de gaz pour faire passer un flux de gaz à travers ledit orifice de pulvérisation de ladite tête de pulvérisation et l'envoyer sur ladite zone de réception, ledit ferrofluide contacté par ledit flux de gaz étant déplacé sur ladite plaque transparente pour former une zone d'impact,
- une caméra disposée sous ladite plaque transparente pour visualiser ladite zone d'impact dudit flux de gaz comprimé sur ladite plaque transparente en prenant une image dudit ferrofluide déplacé par ledit flux de gaz sur ladite plaque transparente, et
- des moyens d'analyse pour analyser ladite image de ladite zone d'impact pour déterminer si ladite zone d'impact est conforme ou non conforme à des spécifications prédéterminées.

Avantageusement, ledit flux de gaz est un flux d'air comprimé.

Avantageusement, ledit ferrofluide est une suspension colloïdale de nanoparticules ferromagnétiques ou ferrimagnétiques dans un solvant ou de l'eau.

Avantageusement, lesdits moyens de génération du flux de gaz comprimé sont adaptés à générer des impulsions de durée réglable, notamment de 50 à 300 ms.

Avantageusement, ledit ensemble d'aimants comporte des aimants et/ou des électro-aimants.

Ces caractéristiques et avantages et d'autres apparaîtront plus clairement au cours de la description détaillée suivante, faite en référence aux dessins joints, donnés à titre d'exemples non limitatifs, et sur lesquels :
la figure 1 est une vue schématique d'un dispositif pour analyser un dispositif de pulvérisation, selon un mode de réalisation avantageux, avant utilisation,
la figure 2 est une vue schématique similaire à celle de la figure 1, en cours d'utilisation,
la figure 3 montre une visualisation de la zone de réception au repos,
la figure 4 montre une visualisation d'une zone d'impact conforme, et
la figure 5 montre une visualisation d'une zone d'impact non-conforme.

Un objectif de l'invention est d'améliorer la qualité du contrôle des dispositifs de pulvérisation. Pour cela, l'invention prévoit d'analyser 100% des dispositifs, sans ralentissement substantiel de la chaine de montage.

De manière standard, chaque dispositif de pulvérisation comprend une tête de pulvérisation 1 pourvue d'un orifice de pulvérisation 2. Généralement, un profil de pulvérisation (non représenté) est prévu en amont dudit orifice de pulvérisation 2 pour générer une forme de spray conique en sortie de l'orifice.

La présente invention prévoit de faire passer un flux de gaz F1, de préférence comprimé, à travers chaque tête de pulvérisation 1, et de diriger ce flux de gaz F1, sortant de l'orifice de pulvérisation 2 avec la forme d'un spray conique, vers une zone de réception 10. Avantageusement, le flux de gaz F1 est un flux d'air comprimé, mais il est entendu que selon l'invention, n'importe quel gaz approprié autre que de l'air pourrait être utilisé.

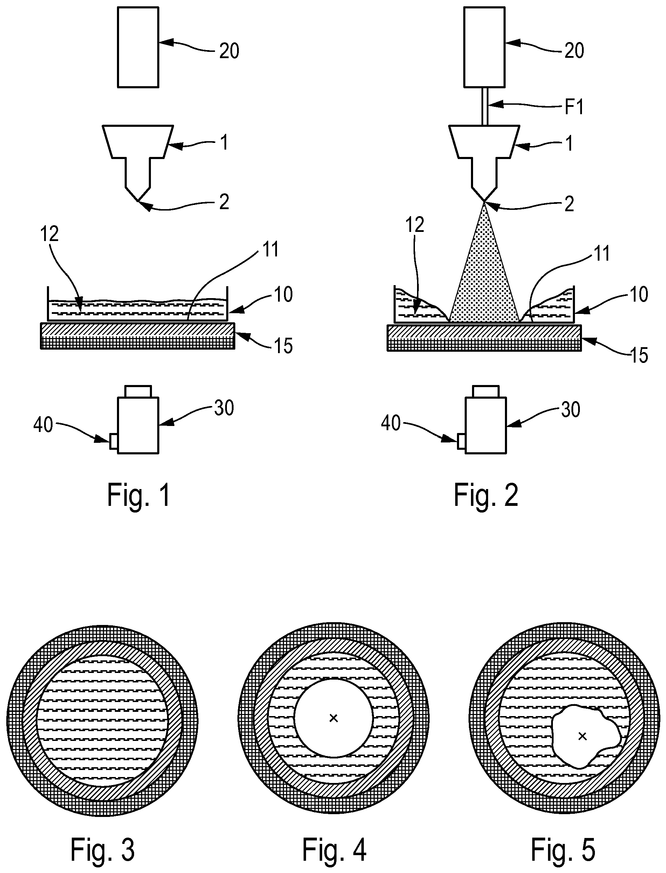

Les figures 1 et 2 montrent un dispositif de test selon un mode de réalisation avantageux.

Dans cet exemple, une tête de pulvérisation 1 est disposée en face d'une zone de réception 10. Des moyens de génération 20 d'un flux de gaz F1 sont prévus pour faire passer un flux de gaz F1 à travers la tête de pulvérisation 1.

La zone de réception 10 forme un réservoir dont le fond est formé par une plaque transparente 11. Ce réservoir contient un ferrofluide 12. Un ensemble d'aimants 15 générant une charge magnétique est disposé sous la plaque transparente 11 pour contraindre le ferrofluide 12 à recouvrir ladite plaque transparente 11. Avantageusement, l'ensemble d'aimants 15 forme un anneau définissant une partie centrale vide à travers laquelle la plaque transparente 11 reste visible. L'ensemble d'aimants 15 peut comporter des aimants et/ou des électro-aimants.

Les ferrofluides sont des suspensions colloïdales de nanoparticules ferromagnétiques ou ferrimagnétiques, typiquement d'une taille de l'ordre de 10 nanomètres, dans un solvant ou de l'eau. Ces liquides deviennent magnétiques lors de l'application d'un champ magnétique extérieur tout en conservant leur stabilité colloïdale. Les ferrofluides sont le plus fréquemment composés de nanoparticules de magnétite (Fe3O4) ou de maghémite (γ-Fe2O3), qui sont tous deux des oxydes de fer.

Lorsque le flux de gaz F1 est envoyé sur la zone de réception 10, le ferrofluide 12 se déforme sous l'effet du flux de gaz et est donc repoussé vers l'extérieur, pour se concentrer au niveau des parois du réservoir. Ce qui n'est pas soufflé par le flux de gaz F1 est maintenu en place par la charge magnétique.

Ainsi, la partie de la plaque transparente 11 dépourvue de ferrofluide 12 après l'envoi du flux de gaz F1 correspond à la zone d'impact dudit flux de gaz F1 sur la zone de réception 10.

Derrière la plaque transparente 11, il est disposé une caméra 30, pour visualiser cette zone d'impact.

Ainsi, le fonctionnement du dispositif de l'exemple des figures 1 et 2 est le suivant.

L'ensemble d'aimants 15 génère une charge magnétique qui force le ferrofluide 12 à recouvrir de manière sensiblement homogène la plaque transparente 11 du réservoir formant la zone de réception 10, comme visible sur la figure 1.

Un flux de gaz F1, notamment d'air comprimé, est alors généré et envoyé à travers la tête de pulvérisation 1 vers la zone de réception 10. La force du flux de gaz F1 déforme le ferrofluide 12 sur la plaque perforée 11 en surmontant la charge magnétique de l'ensemble d'aimants 15. En d'autres termes, la partie du ferrofluide 12 qui est contactée par ledit flux de gaz F1 va être repoussé vers les bords de la plaque 11, comme illustré sur la figure 2.

La caméra 30 prend alors une image de cette plaque 11, avec le ferrofluide 12 ayant été impactée par le flux de gaz F1 formant sur ladite plaque 11 la zone d'impact.

Après chaque utilisation, dès que le flux de gaz F1 cesse, le ferrofluide 12 revient sous l'effet de la charge magnétique dans sa position initiale dans laquelle il recouvre la plaque 11, et on peut relancer quasi-immédiatement un nouveau test.

Pour réaliser les évaluations de conformité, on prévoit une caméra 30 pour visualiser la zone d'impact et des moyens d'analyse 40 pour analyser les visualisations générées par la caméra 30 et ainsi déterminer si la zone d'impact du flux de gaz F1 issu de ladite tête de pulvérisation 1 sur la zone de réception 10 est conforme ou non conforme à des spécifications prédéterminées.

La durée de l'impulsion de gaz F1 est avantageusement réglable, notamment de 50 à 300 ms.

Avantageusement, on peut réaliser plusieurs cycles successifs sur une même tête de pulvérisation, par exemple cinq cycles. La constance ou répétabilité des résultats permet également d'évaluer la conformité de ladite tête de pulvérisation.

Les spécifications prédéterminées peuvent comprendre une étendue planaire prédéterminée de ladite zone d'impact sur ladite zone de réception 10, de sorte que les têtes de pulvérisation 1 pour lesquelles ladite étendue planaire est similaire à ladite étendue planaire prédéterminée sont classées conformes, et les têtes de pulvérisation 1 pour lesquelles ladite étendue planaire est différente de ladite étendue planaire prédéterminée sont classés non conformes. La géométrie, et notamment la symétrie, de la zone d'impact peut aussi être utilisée dans l'évaluation de conformité. D'autres paramètres peuvent aussi être envisagés.

Les moyens d'analyse 40 peuvent comprendre des moyens de mesure de la géométrie de la zone d'impact du flux de gaz F1 sur la zone de réception 10. Par exemple, on détermine le barycentre de la zone d'impact, et on mesure les distances maximale et minimale de ce barycentre du bord de la zone d'impact. La comparaison de ces distances avec des valeurs prédéterminées permet alors d'évaluer la conformité du dispositif testé. Ainsi, l'évaluation de conformité tient compte non seulement de la surface de la zone d'impact, mais aussi de sa géométrie, en particulier sa symétrie. Ceci permet d'établir qu'un spray sortant d'une tête de pulvérisation conforme aura une forme conique acceptable, tant d'un point de vue de l'angle du spray que de sa symétrie.

Eventuellement, des moyens de traitement d'image peuvent être utilisés pour réaliser ce type d'analyse.

Les figures 4 et 5 illustrent chacune une représentation schématique obtenue avec le procédé et le dispositif de l'invention, sur lesquelles il est possible d'évaluer l'étendue planaire et la géométrie, notamment la symétrie, de la zone d'impact. La figure 4 montre une visualisation de la zone d'impact pour un dispositif conforme et la figure 5 montre une telle visualisation pour un dispositif non-conforme.

La présente invention présente de nombreux avantages, et notamment :
- elle permet un contrôle automatisé de conformité sur divers types de dispositif de pulvérisation ;
- elle permet une analyse non destructive desdits dispositifs de pulvérisation ;
- elle permet d'analyser 100% des dispositifs de pulvérisation assemblés sur une chaine de montage, sans ralentissement substantiel de celle-ci ;
- elle permet de réaliser plusieurs tests successifs sur un même dispositif pour évaluer la répétabilité des résultats ;
- elle utilise un montage compact et facilement adaptable ;
- elle utilise des composants simples et standards, donc généralement peu coûteux ;
- elle ne nécessite ni moyens de chauffage, ni caméra thermique, ni moyens de détection thermosensibles ;
- elle permet un traitement d'image robuste, qui peut être réalisé en temps réel ;
- elle assure une bonne répétabilité et une bonne discrimination des dispositifs conformes et non-conformes.

La présente invention a été décrite en référence à un mode de réalisation avantageux, mais il est entendu qu'un homme du métier peut y apporter toutes modifications, sans sortir du cadre de la présente invention tel que défini par les revendications annexées.

## Revendications

1. Procédé d'analyse d'un dispositif de pulvérisation de produit fluide pharmaceutique, comprenant les étapes suivantes :
- fournir une tête de pulvérisation (1) d'un dispositif de pulvérisation de produit fluide pharmaceutique, ladite tête de pulvérisation (1) comportant un orifice de pulvérisation (2),
- fournir une zone de réception (10) formant un réservoir dont le fond est formé par une plaque transparente (11) derrière laquelle est disposée une caméra (30), ledit réservoir contenant un ferrofluide (12),
- fournir, sous ladite plaque transparente (11), un ensemble d'aimants (15) générant une charge magnétique pour contraindre ledit ferrofluide (12) à recouvrir ladite plaque transparente (11),
- faire passer un flux de gaz (F1) à travers ledit orifice de pulvérisation (2) de ladite tête de pulvérisation (1), et l'envoyer sur ladite zone de réception (10), ledit ferrofluide (12) contacté par ledit flux de gaz (F1) étant déplacé sur ladite plaque transparente (11) pour former une zone d'impact,
- visualiser ladite zone d'impact avec ladite caméra (30), et
- analyser avec des moyens d'analyse (40) ladite visualisation de ladite zone d'impact pour déterminer si ladite zone d'impact est conforme ou non conforme à des spécifications prédéterminées.

2. Procédé selon la revendication 1, dans lequel ledit flux de gaz (F1) est un flux de gaz comprimé.

3. Procédé selon la revendication 1 ou 2, dans lequel ledit flux de gaz (F1) est un flux d'air comprimé.

4. Procédé selon l'une quelconque des revendications précédentes, dans lequel ladite étape d'analyser comprend de déterminer la géométrie, notamment la symétrie, de la zone d'impact dudit flux de gaz (F1) sur ladite zone de réception (10).

5. Procédé selon l'une quelconque des revendications précédentes, dans lequel lesdites spécifications prédéterminées comprennent une étendue planaire prédéterminée de la zone d'impact dudit flux de gaz (F1) sur ladite zone de réception (10), de sorte que les têtes de pulvérisation (1) pour lesquelles ladite étendue planaire est similaire à ladite étendue planaire prédéterminée sont classées conformes, et les têtes de pulvérisation (1) pour lesquelles ladite étendue planaire est différente de ladite étendue planaire prédéterminée sont classés non conformes.

6. Procédé selon l'une quelconque des revendications précédentes, dans lequel un cycle d'utilisation comprend les étapes suivantes :
- activer ledit ensemble d'aimants (15) pour générer une charge magnétique pour contraindre ledit ferrofluide (12) à recouvrir ladite plaque transparente (11),
- générer ledit flux de gaz (F1) et l'envoyer à travers ladite tête de pulvérisation (1) sur ladite zone de réception (10), le ferrofluide (12) contacté par ledit flux de gaz (F1) étant repoussé vers les bords de ladite zone de réception (10) pour former une zone d'impact sur ladite plaque transparente (11),
- visualiser ladite zone d'impact en prenant une image de ladite plaque transparente (11) au moyen de ladite caméra (30).

7. Dispositif d'analyse d'un dispositif de pulvérisation de produit fluide pharmaceutique, comprenant :
- une tête de pulvérisation (1) d'un dispositif de pulvérisation de produit fluide pharmaceutique, ladite tête de pulvérisation (1) comportant un orifice de pulvérisation (2),
- une zone de réception (10) formant un réservoir dont le fond est formé par une plaque transparente (11), ledit réservoir contenant un ferrofluide (12),
- un ensemble d'aimants (15) générant une charge magnétique pour contraindre ledit ferrofluide (12) à recouvrir ladite plaque transparente (11),
- des moyens de génération (20) d'un flux de gaz (F1) pour faire passer un flux de gaz (F1) à travers ledit orifice de pulvérisation (2) de ladite tête de pulvérisation (1) et l'envoyer sur ladite zone de réception (10), ledit ferrofluide (12) contacté par ledit flux de gaz (F1) étant déplacé sur ladite plaque transparente (11) pour former une zone d'impact,
- une caméra (30) disposée sous ladite plaque transparente (11) pour visualiser ladite zone d'impact dudit flux de gaz comprimé (F1) sur ladite plaque transparente (11) en prenant une image dudit ferrofluide (12) déplacé par ledit flux de gaz (F1) sur ladite plaque transparente (11), et
- des moyens d'analyse (40) pour analyser ladite image de ladite zone d'impact pour déterminer si ladite zone d'impact est conforme ou non conforme à des spécifications prédéterminées.

8. Dispositif selon la revendication 7, dans lequel ledit flux de gaz (F1) est un flux d'air comprimé.

9. Dispositif selon la revendication 7 ou 8, dans lequel ledit ferrofluide est une suspension colloïdale de nanoparticules ferromagnétiques ou ferrimagnétiques dans un solvant ou de l'eau.

10. Dispositif selon l'une quelconque des revendications 7 à 9, dans lequel lesdits moyens de génération (20) du flux de gaz comprimé (F1) sont adaptés à générer des impulsions de durée réglable, notamment de 50 à 300 ms.

11. Dispositif selon l'une quelconque des revendications 7 à 10, dans lequel ledit ensemble d'aimants (15) comporte des aimants et/ou des électro-aimants.

## Patentansprüche

1. Verfahren zur Analyse einer Sprühvorrichtung für ein pharmazeutisches Fluidprodukt, das folgende Schritte aufweist:
- Bereitstellen eines Sprühkopfes (1) einer Sprühvorrichtung für ein pharmazeutisches Fluidprodukt, wobei der Sprühkopf (1) eine Sprühöffnung (2) aufweist,
- Bereitstellen eines Aufnahmebereichs (10), der einen Behälter bildet, dessen Boden durch eine transparente Platte (11) gebildet ist, hinter der eine Kamera (30) angeordnet ist, wobei der Behälter ein Ferrofluid (12) enthält,
- Bereitstellen, unterhalb der transparenten Platte (11), einer Magnetanordnung (15), die ein Magnetfeld erzeugt, um das Ferrofluid (12) dazu zu veranlassen, die transparente Platte (11) zu bedecken,
- Leiten eines Gasstroms (F1) durch die Sprühöffnung (2) des Sprühkopfes (1) und Senden desselben auf den Aufnahmebereich (10), wobei das durch den Gasstrom (F1) in Kontakt gebrachte Ferrofluid (12) auf der transparenten Platte (11) verschoben wird, um eine Aufprallzone zu bilden,
- Visualisieren der Aufprallzone mittels der Kamera (30), und
- Analysieren mittels Analysemitteln (40) der Visualisierung der Aufprallzone, um zu bestimmen, ob die Aufprallzone vorbestimmten Spezifikationen entspricht oder nicht entspricht.

2. Verfahren nach Anspruch 1, wobei der Gasstrom (F1) ein komprimierter Gasstrom ist.

3. Verfahren nach Anspruch 1 oder 2, wobei der Gasstrom (F1) ein komprimierter Luftstrom ist.

4. Verfahren nach einem der vorhergehenden Ansprüche, wobei der Schritt des Analysierens umfasst, die Geometrie, insbesondere die Symmetrie, der Aufprallzone des Gasstroms (F1) auf dem Aufnahmebereich (10) zu bestimmen.

5. Verfahren nach einem der vorhergehenden Ansprüche, wobei die vorbestimmten Spezifikationen eine vorbestimmte planare Ausdehnung der Aufprallzone des Gasstroms (F1) auf dem Aufnahmebereich (10) umfassen, so dass die Sprühköpfe (1), bei denen die planare Ausdehnung der vorbestimmten planaren Ausdehnung ähnlich ist, als konform klassifiziert werden, und die Sprühköpfe (1), bei denen die planare Ausdehnung von der vorbestimmten planaren Ausdehnung verschieden ist, als nicht konform klassifiziert werden.

6. Verfahren nach einem der vorhergehenden Ansprüche, wobei ein Nutzungszyklus die folgenden Schritte umfasst:
- Aktivieren der Magnetanordnung (15), um ein Magnetfeld zu erzeugen, um das Ferrofluid (12) dazu zu veranlassen, die transparente Platte (11) zu bedecken,
- Erzeugen des Gasstroms (F1) und Senden desselben durch den Sprühkopf (1) auf den Aufnahmebereich (10), wobei das durch den Gasstrom (F1) in Kontakt gebrachte Ferrofluid (12) zu den Rändern des Aufnahmebereichs (10) verdrängt wird, um eine Aufprallzone auf der transparenten Platte (11) zu bilden,
- Visualisieren der Aufprallzone durch Aufnehmen eines Bildes der transparenten Platte (11) mittels der Kamera (30).

7. Vorrichtung zur Analyse einer Sprühvorrichtung für ein pharmazeutisches Fluidprodukt mit
- einem Sprühkopf (1) einer Sprühvorrichtung für ein pharmazeutisches Fluidprodukt, wobei der Sprühkopf (1) eine Sprühöffnung (2) aufweist,
- einem Aufnahmebereich (10), der einen Behälter bildet, dessen Boden durch eine transparente Platte (11) gebildet ist, wobei der Behälter ein Ferrofluid (12) enthält,
- einer Magnetanordnung (15), die ein Magnetfeld erzeugt, um das Ferrofluid (12) dazu zu veranlassen, die transparente Platte (11) zu bedecken,
- Mitteln zur Erzeugung (20) eines Gasstroms (F1), um einen Gasstrom (F1) durch die Sprühöffnung (2) des Sprühkopfes (1) zu leiten und diesen auf den Aufnahmebereich (10) zu senden, wobei das durch den Gasstrom (F1) in Kontakt gebrachte Ferrofluid (12) auf der transparenten Platte (11) verschoben wird, um eine Aufprallzone zu bilden,
- einer Kamera (30), die unterhalb der transparenten Platte (11) angeordnet ist, um die Aufprallzone des komprimierten Gasstroms (F1) auf der transparenten Platte (11) zu visualisieren, indem ein Bild des durch den Gasstrom (F1) auf der transparenten Platte (11) verschobenen Ferrofluids (12) aufgenommen wird, und
- Analysemitteln (40), um das Bild der Aufprallzone zu analysieren, um zu bestimmen, ob die Aufprallzone vorbestimmten Spezifikationen entspricht oder nicht entspricht.

8. Vorrichtung nach Anspruch 7, wobei der Gasstrom (F1) ein komprimierter Luftstrom ist.

9. Vorrichtung nach Anspruch 7 oder 8, wobei das Ferrofluid eine kolloidale Suspension von ferromagnetischen oder ferrimagnetischen Nanopartikeln in einem Lösungsmittel oder in Wasser ist.

10. Vorrichtung nach einem der Ansprüche 7 bis 9, wobei die Mittel zur Erzeugung (20) des komprimierten Gasstroms (F1) dazu eingerichtet sind, Impulse einstellbarer Dauer, insbesondere von 50 bis 300 ms, zu erzeugen.

11. Vorrichtung nach einem der Ansprüche 7 bis 10, wobei die Magnetanordnung (15) Magnete und/oder Elektromagnete umfasst.

## Claims

1. A method for analysing a device for spraying a pharmaceutical fluid product, comprising the following steps:
- providing a spraying head (1) for a device for spraying a pharmaceutical fluid product, said spraying head (1) comprising a spraying opening (2),
- providing a receiving zone (10) forming a container the bottom of which is formed by a transparent plate (11) behind which a camera (30) is arranged, said container containing a ferrofluid (12),
- providing, under said transparent plate (11), a set of magnets (15) generating a magnetic charge to force said ferrofluid (12) to cover said transparent plate (11),
- passing a stream of gas (F1) through said spraying opening (2) of said spraying head (1), and sending it over said receiving zone (10), said ferrofluid (12) contacted by said stream of gas (F1) being moved over said transparent plate (11) to form an impact zone,
- viewing said impact zone with said camera (30), and
- means for analysing (40), for analysis said view of said impact zone in order to determine whether or not said impact zone complies with predetermined specifications.

2. The method as claimed in claim 1, wherein said stream of gas (F1) is a stream of compressed gas.

3. The method as claimed in claim 1 or claim 2, wherein said stream of gas (F1) is a compressed air stream.

4. The method as claimed in any one of the preceding claims, wherein said step for analysing comprises determining the geometry, in particular the symmetry, of the impact zone for said stream of gas (F1) over said receiving zone (10).

5. The method as claimed in any one of the preceding claims, wherein said predetermined specifications comprise a predetermined planar extent of the impact zone for said stream of gas (F1) over said receiving zone (10), in a manner such that the spraying heads (1) for which said planar extent is similar to said predetermined planar extent are classified as compliant, and the spraying heads (1) for which said planar extent is different from said predetermined planar extent are classified as non-compliant.

6. The method as claimed in any one of the preceding claims, wherein an operating cycle comprises the following steps:
- activating said set of magnets (15) to generate a magnetic charge to force said ferrofluid (12) to cover said transparent plate (11),
- generating said stream of gas (F1) and sending it through said spraying head (1) over said receiving zone (10), the ferrofluid (12) contacted by said stream of gas (F1) being pushed back towards the edges of said receiving zone (10) to form an impact zone over said transparent plate (11),
- viewing said impact zone by taking an image of said transparent plate (11) by means of said camera (30).

7. A device for analysing a device for spraying a pharmaceutical fluid product, comprising:
- a spraying head (1) of a device for spraying a pharmaceutical fluid product, said spraying head (1) comprising a spraying opening (2),
- a receiving zone (10) forming a container the bottom of which is formed by a transparent plate (11), said container containing a ferrofluid (12),
- a set of magnets (15) generating a magnetic charge to force said ferrofluid (12) to cover said transparent plate (11),
- means (20) for generating a stream of gas (F1) in order to pass a stream of gas (F1) through said spraying opening (2) of said spraying head (1) and sending it over said receiving zone (10), said ferrofluid (12) contacted by said stream of gas (F1) being moved over said transparent plate (11) to form an impact zone,
- a camera (30) arranged under said transparent plate (11) for viewing said impact zone of said stream of compressed gas (F1) over said transparent plate (11) by taking an image of said ferrofluid (12) moved by said stream of gas (F1) over said transparent plate (11), and
- means for analysing (40), for analysing said image of said impact zone in order to determine whether or not said impact zone complies with predetermined specifications.

8. The device as claimed in claim 7, wherein said stream of gas (F1) is a compressed air stream.

9. The device as claimed in claim 7 or claim 8, wherein said ferrofluid is a colloidal suspension of ferromagnetic or ferrimagnetic nanoparticles in a solvent or water.

10. The device as claimed in any one of claims 7 to 9, wherein said means (50) for generating a stream of compressed gas (F1) are adapted to generate pulses of adjustable duration, in particular from 50 to 300 ms.

11. The device as claimed in any one of claims 7 to 10, wherein said magnet assembly (15) comprises magnets and/or electromagnets.
